# EUROPÄISCHE PATENTSCHRIFT

(11) **EP 1 519 699 B1**
(45) Veröffentlichungstag und Bekanntmachung des Hinweises auf die Patenterteilung: **28.06.2006**
(21) Anmeldenummer: 03762349.3
(22) Anmeldetag: 08.07.2003
(51) Int. Cl.: A61F 5/058

(54) **EINRICHTUNG ZUR STÜTZUNG UND STABILISIERUNG EINES VERLETZTEN ODER VERLETZTEN KÖRPERTEILS SOWIE VERFAHREN ZU DESSEN HERSTELLUNG**
DEVICE FOR SUPPORTING AND STABILISING AN INJURED PERSON OR AN INJURED LIMB, AND METHOD FOR PRODUCING SAID DEVICE
DISPOSITIF POUR SOUTENIR ET STABILISER UN BLESSE OU UNE PARTIE DU CORPS BLESSEE, ET PROCEDE DE REALISATION D'UN TEL DISPOSITIF

(30) Priorität: 09.07.2002 AT 10292002
(43) Veröffentlichungstag der Anmeldung: 06.04.2005
(73) Patentinhaber: Kohlbrat & Bunz Gesellschaft m.b.H, 5550 Radstadt (AT)
(72) Erfinder: RUGFELT, Hakan, SE-239 41 Falsterbo (SE)
(74) Vertreter: Torggler, Paul Norbert
(86) Internationale Anmeldenummer: PCT/AT2003/000190
(87) Internationale Veröffentlichungsnummer: WO 2004/004608

(56) Entgegenhaltungen:
- WO-A-01/30280
- CH-A- 661 204
- NL-A- 7 101 392
- US-A- 3 745 998
- US-A- 5 154 185

## Beschreibung

Die Erfindung betrifft eine Einrichtung zur Stützung und Stabilisierung eines Verletzten oder verletzten Körperteils, mit einem flexiblen, um den Verletzten oder den Körperteil festlegbaren Folienelement, das einen luftdichten, evakuierbaren Innenraum einschließt, in dem zwei Einsatzkörper vorgesehen sind, die jeweils aus zwei luftdurchlässigen, flexiblen Materialbahnen gebildet sind, wobei jeder Einsatzkörper durch mehrere parallele Nähte zwischen den Materialbahnen in Kammern unterteilt ist, die lose Partikel enthalten, wobei die Nähte an beiden Einsatzkörpern zueinander so versetzt sind, daß die Partikel einander zu einer im wesentlichen gleichmäßig dicken Partikelschicht ergänzen sowie ein Verfahren zur Herstellung eines Einsatzkörpers.

Als Vakuummatratzen, Vakuumwesten und Vakuumschienen bezeichnete Rettungs-und Transporteinrichtungen weisen eine Hülle aus einer luftdichten Kunststoffolie und eine Füllung aus einem Kunststoffgranulat, insbesondere aus geschäumten Polystyrolkugeln, auf und können nach der Anpassung und Fixierung an einem ruhig zu stellenden Verletzten oder verletzten Körperteil mittels einer Saugpumpe evakuiert werden. Dies führt zu einer dichten Packung des eingefüllten Granulats und somit zu einer Versteifung des flexiblen Elements, das auf diese Weise eine im wesentlichen starre Hülle oder Manschette des Körperteils bildet.

Eine Einrichtung dieser Art ist der WO 01/30280 zu entnehmen. Sie zeigt in einer Ausführung eine Unterteilung des Innenraums durch vier luftdurchlässige Materialbahnen in fünf Fächer, wobei die Partikel enthaltenden Kammern im zweiten und im vierten Fach vorgesehen sind. Hiefür sind die Materialbahnen paarweise miteinander durch parallele Verbindungsnähte verbunden, wobei jede Verbindungsnaht zweier Materialbahnen jeweils um die halbe Kammerbreite zu einer Verbindungsnaht der beiden anderen Materialbahnen versetzt ist, sodaß die beiden Reihen von Kammern einander überlappen und jeweils eine Verbindungsnaht im Bereich eines Kammerscheitels liegt. Die Überlappung der Kammern führt trotz fehlender Partikel im Bereich der Verbindungsnähte zu einer annähernd gleichmäßigen Verteilung der Füllung, da bei evakuiertem Innenraum einander die Partikel benachbarter Kammern zu einer im wesentlichen gleichmäßigen Schicht ergänzen. Bei der Herstellung dieser Ausführung werden zuerst zwei aus je zwei Materialbahnen bestehenden Einsatzkörper hergestellt und mit den Partikeln gefüllt. Anschließend werden die Einsatzkörper jeweils entlang ihres Umfangs im Randbereich einer Folie befestigt und schließlich die beiden Folien entlang ihrer Ränder verbunden.

Die Einrichtung nach der WO 01/30280 ist hauptsächlich als Oberkörperstütz- und - stabilisierungsweste ausgebildet, in deren Einsatzkörpern die Kammern in Umfangsrichtung, bei sitzender Position also horizontal um den Oberkörper verlaufen. In Längsrichtung der Kammern wird die Beweglichkeit der Partikel nur durch die geringe Kammernbreite verringert. Dennoch ist es meist erforderlich, die Partikel vor dem Anlegen an den Verletzten so gut es geht händisch zu verteilen, was Zeit kostet. Im evakuierten Zustand ist die Stabilität der Vorrichtung in Längsrichtung der Kammern gut, in Längsrichtung des Verletzten hingegen schlechter, da die Partikelpackung oftmals durch die Knickzonen bildenden Querstege unterbrochen ist.

Die CH 661 204 zeigt eine Stabilisierungsvorrichtung für den gesamten Körper, deren Kammern in Richtung der Wirbelsäule verlaufen. Die oben beschriebenen Nachteile treten daher jeweils um 90° versetzt auf. Die losen Partikel rutschen bei der Handhabung in den Kopf- oder Fußbereich, und die Querstabilität ist schlechter. Hiezu kommt noch ein weiterer, für den Verletzten nachteiliger Effekt, nämlich die beim Evakuieren durch die Verdichtung der Partikelpackung in Richtung der Wirbelsäule beträchtliche Längenschrumpfung im Zentimeterbereich, die gefährliche Kräfte auf die verletzte Wirbelsäule ausübt.

Die Erfindung hat es sich nun zur Aufgabe gestellt, eine Vorrichtung der eingangs genannten Art zu schaffen, die verringerte Längenschrumpfung und eine verbesserte Stabilität in Längs- und in Querrichtung aufweist. Erreicht wird dies dadurch, daß die Nähte an jedem Einsatzkörper einander kreuzen, und die Nähte des einen Einsatzkörpers zu denen des zweiten versetzt sind. Auf diese Weise werden in alle Richtungen durchgehende Kammern vermieden, und die schuppen- bzw. dachziegelartige Überlappung der Kammern stellt die Stabilität der evakuierten Vorrichtung in Längs- und Querrichtung sicher. Ebenso ist durch die einander kreuzenden Nähte die Schrumpfung in diese beiden Richtungen wesentlich verringert, da Länge und Breite der Kammern klein sind.

Insbesondere kreuzen die Nähte einander rechtwinklig, könnten aber auch jeweils Dreiecke bilden. Bevorzugt sind die Nahtraster so versetzt, dass jeder Kreuzungspunkt der Nähte an einem Einsatzkörper in der Mitte eines durch die Nähte gebildeten Rasterfeldes am zweiten Einsatzkörper liegt.

In einer weiteren bevorzugten Ausführung mit einander rechtwinklig kreuzenden Nähten ist vorgesehen, daß die Länge und die Breite jedes Einsatzkörpers in eine ungerade Anzahl von Einheiten unterteilt sind, und der Abstand zwischen je zwei Nähten jeweils zwei Einheiten entspricht, wobei jeweils eine äußerste Naht an beiden Einsatzkörpern eine Einheit vom Rand entfernt ist. Auf diese Weise wird ein nicht zentrierter Raster von Nähten erzielt, der es erlaubt, zwei gleichartige Einsatzkörper vorzusehen, wobei einer der beiden Einsatzkörper in der Ebene um 180° gedreht angeordnet wird.

Eine zweite Ausführung der Einrichtung sieht vor, daß die Länge und die Breite jedes Einsatzkörpers in eine gerade Anzahl von Einheiten unterteilt sind, und der Abstand zwischen je zwei Nähten jeweils zwei Einheiten entspricht, wobei alle äußersten Nähte an einem Einsatzkörper je eine Einheit und am anderen Einsatzkörper je zwei Einheiten vom Rand entfernt sind. Hier sind zwei unterschiedlich abgenähte Einsatzkörper vorgesehen, die beide zentrierte Raster von Nähten aufweisen, wobei ein Einsatzkörper rundum kleinere Randkammern aufweist.

In beiden Fällen werden die beiden Einsatzkörper deckend aufeinandergelegt, und jeder Kreuzungspunkt von Nähten des einen Einsatzkörpers liegt im Zentrum eines von den Nähten begrenzten Feldes des anderen Einsatzkörpers.

Ein derartiger Einsatzkörper kann erfindungsgemäß dadurch hergestellt werden, daß zwei luftdurchlässige, flexible Materialbahnen an zwei einander gegenüberliegenden ersten Rändern, durch zu den ersten Rändern parallele erste Nähte, und an einem der beiden zweiten Ränder miteinander verbunden werden, und daß die gebildeten Kanäle abwechselnd mit Partikel befüllt und durch zum ersten zweiten Rand parallele zweite Nähte in Kammern unterteilt werden, bis der zweite zweite Rand erreicht ist.

Nachstehend wird nun die Erfindung an Hand der Figuren der beiliegenden Zeichnung näher beschrieben, ohne darauf beschränkt zu sein. Es zeigen
- Fig. 1: eine schematische Draufsicht auf eine erste Ausführung einer erfindungsgemäßen Einrichtung ohne Deckfolie,
- Fig. 2: einen Schnitt nach der Linie II-II der Fig. 1,
- Fig. 3: eine schematische Draufsicht auf eine zweite Ausführung, ebenfalls ohne Deckfolie, und
- Fig. 4: einen Schnitt nach der Linie IV-IV der Fig. 3.

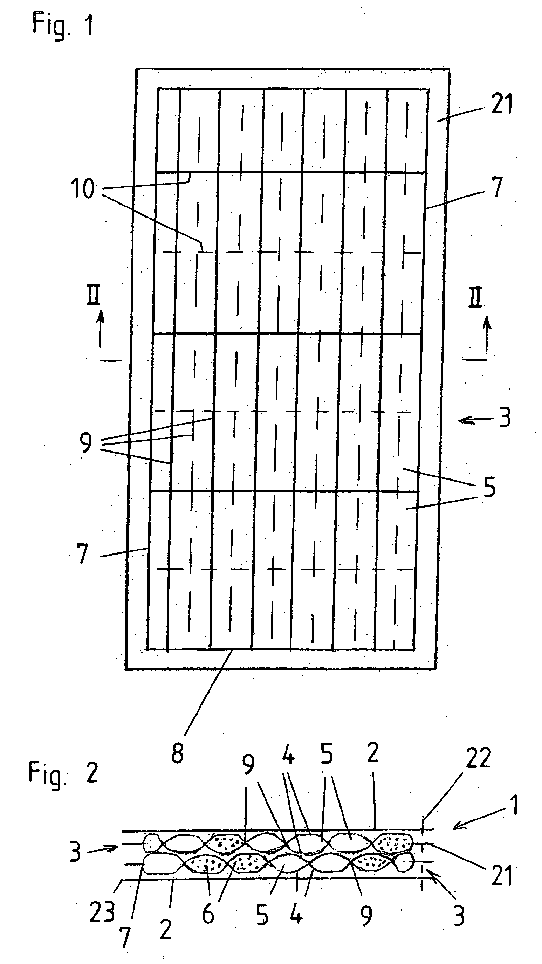

Eine Einrichtung zur Stabilisierung bzw. Stützung eines Verletzten weist ein Folienelement 1 auf, das aus zwei luftdichten Folien 2 zusammengesetzt ist, die beispielsweise aus einem Polyuvinylchlorid oder einem Polyurethan bestehen. Die beiden Folien sind längs ihrer Ränder 23 miteinander verschweißt, was in Fig. 2 und 4 aus Gründen der Übersichtlichkeit durch die strichlierten Linien 22 angedeutet ist. Die am Verletzten zur Anlage kommende Folie 2 ist glatt, und auf der Außenfolie 2 kann, wenn diese aus Polyurethan besteht, ein versteifendes, nicht gezeigtes Folienstück fixiert sein, an dem Ein- und Austrittsschlitze für Befestigungsgurte vorgesehen sind. Alternativ können für die Fixierung der Befestigungsgurte auf der Außenfolie 2 Folienstreifen aufgebracht sein, in denen Ösen mit Mittelsteg angeordnet sind. In der Außenfolie 2 ist ein Ventil angeordnet, durch das Luft aus dem Partikel 6, beispielsweise Kunststoffkugeln, aus geschäumtem Polystyrol oder geschäumtem Polypropylen enthaltenden Innenraum abgesaugt werden kann. Das vor der Anwendung weiche, flache Folienelement 1 wird aus dem flachen Zustand um den Verletzten hochgezogen und mit Hilfe der Gurte beispielsweise am Kopf, Rumpf und Oberschenkeln fixiert. Wird nunmehr die Luft abgesaugt, so versteift sich das Folienelement in der an die Körperform angepaßten Gestalt, da den Partikein 6 der Bewegungsraum entzogen ist und sie durch den äußeren Luftdruck gegeneinander gepreßt sind.

Der Innenraum ist durch vier flexible, luftdurchlässige Materialbahnen 4 unterteilt, wobei aus je zwei Materialbahnen 4 ein Einsatzkörper 3 gebildet ist, in dem die Partikel 6 in Kammern 5 enthalten sind. Die Kammern 5 sind durch einander kreuzende Nähte 9, 10 begrenzt, die die beiden Materialbahnen 4 jedes der beiden Einsatzkörper 3 verbinden. Ein umlaufender Randstreifen 21 jedes Einsatzkörpers 3 dient zur Fixierung zwischen den beiden Folien 2. Die luftdurchlässigen Materialbahnen 4 sind bevorzugt aus einem Gewebe aus Polypropylenfasern hergestellt. Die beiden Einsatzkörper 3 sind so angeordnet, daß die einander kreuzenden Nähte 9 und 10 sowohl in der Längs- als auch in der Querrichtung jeweils um den halben Abstand versetzt sind. Die Nähte 9, 10 des unteren Einsatzkörpers 3 sind strichliert dargestellt. Wie aus den Fig. 2 und 4 ersichtlich, liegt die Mitte oder der Scheitel jeder Kammer 5 über einem Kreuzungspunkt der Nähte 9, 10 des unteren Einsatzkörpers 3. Somit gleicht sich die Höhe der Partikelfüllung in den beiden Einsatzkörpern 3 im wesentlichen aus, da ein Maximum in jedem Scheitel einer Kammer 5 über jedem Kreuzungspunkt der Nähte liegt, in dem die Partikel fehlen.

In der Ausführung nach Fig. 1 und 2 können zwei identische Einsatzkörper 3 verwendet werden, wenn deren Länge und deren Breite in eine gleiche oder unterschiedliche ungerade Anzahl von Einheiten unterteilt werden, beispielsweise 7 Längen- und 13 Breiteneinheiten und der Abstand zwischen je zwei Nähten 9 bzw. 10 jeweils zwei der entsprechenden Einheiten beträgt. Aufgrund der ungeraden Anzahl von Einheiten ist zwangsläufig eine der beiden Nähte 9 im Abstand einer Breiteneinheit vom Längsrand 7, und die zweite im Abstand zweier Breiteneinheiten vom anderen Längsrand 7. Gleiches gilt für die Nähte 10, deren äußerste von den Querrändern 8 eine bzw. zwei Längeneinheiten entfernt sind. Ein Einsatzkörper 3 wird in der Ebene um 180° gedreht (oder um seine Längs- und seine Quermittelachse gewendet), wodurch die in Fig. 1 gezeigte Anordnung der versetzten Nähte erzielt wird.

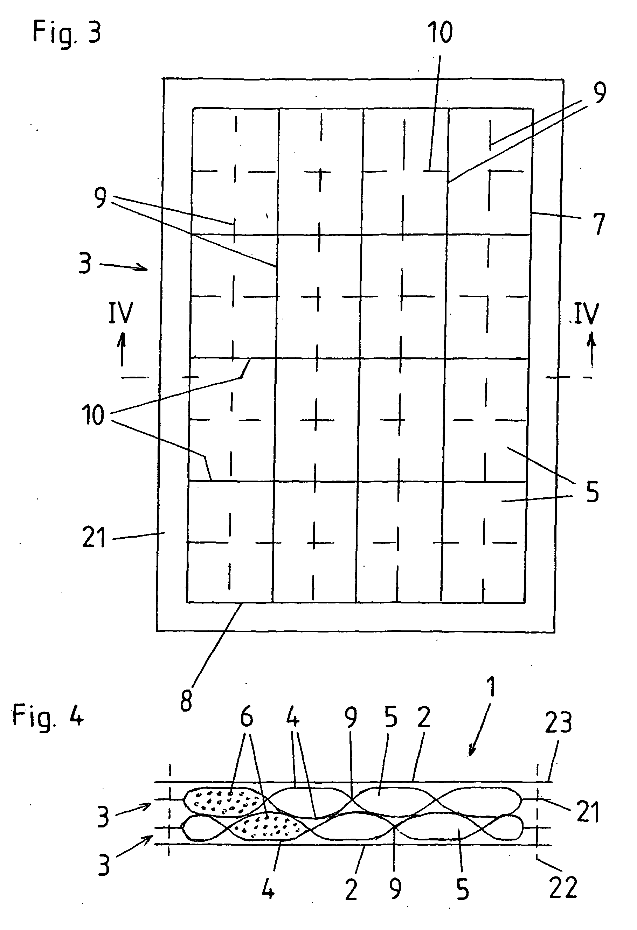

In der Ausführung nach Fig. 3 und 4 sind zwei unterschiedlich genähte Einsatzkörper vorgesehen, wobei Länge und Breite in eine gerade Anzahl von Einheiten, beispielsweise je 8, geteilt sind. Der Abstand zwischen den Nähten 9 oder 10 beträgt wiederum zwei Längen- oder zwei Breiteneinheiten. Unterschiedlich sind die Abstände der äußersten Nähte 9, 10 zu den Längs- und Querrändern 7 und 8, die bei den in Fig. 3 sichtbaren Einsatzkörpern 3 jeweils zwei Einheiten und bei den unteren Einsatzkörpern 3 jeweils rundum eine Einheit betragen.

Die Herstellung eines Einsatzkörpers 3 erfolgt insbesondere so, daß zwei Materialbahnen 4 entsprechender Größe entlang ihrer ersten (Längs-) Ränder 7 und entlang eines zweiten (Quer-) Randes 8 miteinander verbunden, und durch die (Längs-) Nähte 9 in Kanäle unterteilt werden. Anschließend werden Partikel 6 bis zu einer vorgegebenen Höhe (zwei Einheiten) eingefüllt und eine (Quer-) Naht 10 wird gesetzt. Nach der nächsten Füllung wird die nächste (Quer-) Naht gefertigt, usw. bis der andere (Quer-) Rand 8 erreicht ist.

Zwei Einsatzkörper 3 werden übereinander auf eine Folie 2 gelegt und zusammen mit dieser und einer Deckfolie 2 entlang der Randstreifen 21, 23 verschweißt.

## Patentansprüche

1. Einrichtung zur Stützung und Stabilisierung eines Verletzten oder verletzten Körperteils, mit einem flexiblen, um den Verletzten oder den Körperteil festlegbaren Folienelement (1), das einen luftdichten, evakuierbaren Innenraum einschließt, in dem zwei aufeinander liegende Einsatzkörper (3) vorgesehen sind, die jeweils aus zwei luftdurchlässigen, flexiblen Materialbahnen (4) gebildet sind, wobei jeder Einsatzkörper (3) durch mehrere parallele Nähte (9, 10) zwischen den Materialbahnen (4) in Kammern (5) unterteilt ist, die lose Partikel (6) enthalten, wobei die Nähte (9, 10) an beiden Einsatzkörpern (3) zueinander so versetzt sind, daß die Partikel (6) einander zu einer im wesentlichen gleichmäßig dicken Partikelschicht ergänzen, **dadurch gekennzeichnet, daß** die Nähte (9, 10) an jedem Einsatzkörper (3) einander kreuzen und einen Nahtraster bilden, und daß die beiden Nahtraster zueinander versetzt sind.

2. Einrichtung nach Anspruch 1, **dadurch gekennzeichnet, daß** die Nähte (9, 10) einander rechtwinklig kreuzen.

3. Einrichtung nach Anspruch 1 oder 2, **dadurch gekennzeichnet, daß** die beiden Nahtraster so versetzt sind, daß jeder Kreuzungspunkt der Nähte (9, 10) an einem Einsatzkörper (3) in der Mitte eines durch die Nähte (9, 10) gebildeten Rasterfeldes am zweiten Einsatzkörper (3) liegt.

4. Einrichtung nach Anspruch 2 oder 3, **dadurch gekennzeichnet, daß** die Länge und die Breite jedes Einsatzkörpers (3) in eine ungerade Anzahl von Einheiten unterteilt sind, und der Abstand zwischen je zwei Nähten (9 bzw. 10) jeweils zwei Einheiten entspricht, wobei jeweils eine äußerste Naht (9 bzw. 10) an beiden Einsatzkörpern (3) eine Einheit vom Rand (7, 8) entfernt ist.

5. Einrichtung nach Anspruch 2 oder 3, **dadurch gekennzeichnet, daß** die Länge und die Breite jedes Einsatzkörpers (3) in eine gerade Anzahl von Einheiten unterteilt sind, und der Abstand zwischen je zwei Nähten (9 bzw. 10) jeweils zwei Einheiten entspricht, wobei alle äußersten Nähte (9 bzw. 10) an einem Einsatzkörper (3) je eine Einheit und am anderen Einsatzkörper (3) je zwei Einheiten vom Rand (7 bzw. 8) entfernt sind.

6. Verfahren zur Herstellung eines Einsatzkörpers für eine Einrichtung zur Stützung und Stabilisierung eines Verletzten oder verletzten Körperteils mittels eines flexiblen, um den Verletzten oder den Körperteil festlegbaren Folienelernentes (1), das einen luftdichten, evakuierbaren Innenraum einschließt, **dadurch gekennzeichnet, daß** zwei luftdurchlässige, flexible Materialbahnen (4) an zwei einander gegenüberliegenden ersten Rändern (7), durch zu den ersten Rändern (7) parallele erste Nähte (9), und an einem der beiden zweiten Ränder (8) miteinander verbunden werden, und daß die gebildeten Kanäle abwechselnd mit Partikel (6) befüllt und durch zum ersten zweiten Rand (8) parallele zweite Nähte (10) in Kammern (5) unterteilt werden, bis der zweite zweite Rand (8) erreicht ist.

## Claims

1. A device for supporting and stabilizing an injured person or injured body part, comprising a flexible film element (1) to be secured to the injured person or body part and enclosing an airtight evacuable inner space, in which two insert bodies (3) lying in one another are disposed, each being formed from two air-permeable, flexible lengths (4) of material, whereby each insert body (3) is divided by a plurality of seams (9, 1D) parallel to each other into chambers each containing loose particles, whereby the seams (9, 10) on each insert body (3) are offset such that the particles complement one another to form a substantially uniformly thick layer, **characterised in that** the seams (9, 10) of each insert body (3) intersect one another forming a respective grid of seams, and that the grids of seams are offset with respect to one another.

2. The device according to claims 1, **characterised in that** said seams (9, 10) intersect one another at right angles.

3. The device according to claim 1 or 2, **characterised in that** said two grids of seams are offset with respect to one another such that each intersecting point of said seams (9, 10) on one insert body (3) lies in a center of a grid area formed between said seams (9, 10) on the other insert body (3).

4. The device according to claim 2 or 3, **characterised in that** the length and the width of each insert body (3) are divided into an uneven number of units, and the distance between two respective seams (9 or 10) corresponds in each case to two units, whereby one outermost seam (9 or 10) on each insert body (3) is spaced by one unit from an edge (7, 8) thereof.

5. The device according to claims 2 or 3, **characterised in that** the length and the width of each insert body (3) are divided into an even number of units, and the distance between two respective seams (9 or 10) corresponds in each case to two units, whereby all outermost seams (9 or 10) are spaced by one unit on one insert body (3) and by two units on the other insert body (3) from an edge (7 or 8) thereof.

6. A method for producing an insert body for a device for supporting and stabilizing an injured person or injured body part by way of a flexible film element (1) to be secured around the injured person or body part and enclosing an airtight evacuable inner space, **characterised in that** two air-permeable, flexible lengths (4) of material are connected to one another on two mutually opposite first edges (7), by way of first seams (9) extending parallel to the first edges (7), and on one of two second edges (8), and that the so formed channels alternately are filled with particles and divided into chambers (5) by second seams (10) extending parallel to the first second edge (8) until the second second edge (8) is reached.

## Revendications

1. Installation pour soutenir et stabiliser un blessé ou une partie du corps blessée, comportant un élément en feuille (1) souple qui peut être fixé autour du blessé ou de la partie du corps et enferme un espace intérieur étanche à l'air , pouvant être évacué, moyennant quoi sont prévus deux corps d'insertion (3) reposant l'un sur l'autre qui sont formés par deux bandes de matière (4) souples, perméables à l'air, dans lequel chaque corps d'insertion (3) est divisé par plusieurs joints parallèles (9, 10) entre les bandes de matière (4) en des compartiments (5) qui contiennent des particules libres (6), les joints (9, 10) sur les deux corps d'insertion (3) étant décalés les uns par rapport aux autres, de telle sorte que les particules (6) se complètent entre elles pour former une couche de particules d'épaisseur essentiellement régulière, **caractérisée en ce que** les joints (9, 10) se croisent entre eux sur chaque corps d'insertion (3) et forment un réseau de joints et **en ce que** les deux réseaux de joints sont décalés l'un par rapport à l'autre.

2. Installation selon la revendication 1,
**caractérisée en ce que** les joints (9, 10) se croisent à angle droit.

3. Installation selon la revendication 1 ou 2, **caractérisée en ce que** les deux réseaux de joints sont décalés de telle sorte que chaque point de croisement des joints (9, 10) sur un corps d'insertion (3) est situé au milieu d'une zonee de réseau formée par les joints (9, 10) sur le deuxième corps d'insertion (3).

4. Installation selon la revendication 2 ou 3, **caractérisée en ce que** la longueur et la largeur de chaque corps d'insertion (3) sont divisées en un nombre impair d'unités, et l'écart entre deux joints (9 ou 10) correspond chaque fois à deux unités, dans laquelle un joint extérieur (9 ou 10) sur les deux corps d'insertion (3) est distant du bord (7, 8) d'une unité.

5. installation selon la revendication 2 ou 3, **caractérisée en ce que** la longueur et la largeur de chaque corps d'insertion (3) sont divisées en un nombre pair d'unités, et l'écart entre deux joints (9 ou 10) correspond chaque fois à deux unités, dans laquelle tous les joints extérieurs (9 ou 10) sont distants du bord (7 ou 8) d'une unité sur un corps d'insertion (3) et de deux unités sur l'autre corps d'insertion (3).

6. Procédé de fabrication d'un corps d'insertion pour une installation de soutien et de stabilisation d'un blessé ou d'une partie du corps blessée au moyen d'un élément en feuille (1) souple, qui peut être fixé autour du blessé ou de la partie du corps et enferme un espace intérieur étanche à l'air, pouvant être évacué, **caractérisé en ce que** deux bandes de matière (4) souples, perméables à l'air sont reliées entre elles sur deux premiers bords (7) opposés l'un à l'autre par des premiers joints (9) parallèles aux premiers bords (7) et sur l'un des deux seconds bords (8) et **en ce que** les canaux formés sont remplis alternativement de particules (6) et sont divisés en compartiments (5) par des seconds joints (10) parallèles au premier second bord (8) jusqu'à ce que le second second bord (8) soit atteint.
